# EUROPEAN PATENT SPECIFICATION

(11) **EP 2 219 779 B1**
(45) Date of publication and mention of the grant of the patent: **30.05.2012**
(21) Application number: 07866763.1
(22) Date of filing: 19.11.2007
(51) Int. Cl.: B01J 19/24, C07C 29/62, C07C 31/36, C07D 301/26, C07C 31/42

(54) **CONVERSION OF GLYCERINE TO DICHLOROHYDRINS AND EPICHLOROHYDRIN**
UMWANDLUNG VON GLYCERIN IN DICHLORHYDRINE UND EPICHLORHYDRIN
CONVERSION DU GLYCÉROL EN DICHLORHYDRINES ET ÉPICHLORHYDRINE

(43) Date of publication of application: 25.08.2010
(73) Proprietor: Conser S.P.A., 00144 Rome (IT)
(72) Inventor: CASSARINO, Salvatore, I-00186 Roma (IT); SIMOLA, Flavio, I-00015 Monterotondo (RM) (IT)
(74) Representative: Sarpi, Maurizio
(86) International application number: PCT/IT2007/000810
(87) International publication number: WO 2009/066327

(56) References cited:
- WO-A-2005/021476
- WO-A-2006/020234
- US-A- 4 607 086
- DATABASE WPI Week 200425 Derwent Publications Ltd., London, GB; AN 2004-260557 XP002481679 & JP 2003 342213 A (TOA GOSEI CHEM IND LTD) 3 December 2003 (2003-12-03)

## Description

The present invention relates to a process for the production of dichlorohydrin by catalyzed hydrochlorination of glycerine where the reaction is performed in at least two subsequent stages operating at different pressures with both vapor and liquid recycle.

### BACKGROUND OF THE INVENTION

The present invention relates to a continuous process for the production of dichlorohydrins (DCH) either of the 1,3 or of the 2,3 types by the catalyzed reaction of glycerine (GLY) with hydrogen chloride (HCl).

Epichlorohydrin (EPI) is produced by dehydrochlorination of the DCH by means of an aqueous solution of alkali.

The most worldwide used technology for the production of EPI is based on a process comprising:
■ high temperature chlorination of propylene to allyl chloride
■ formation of DCH by hydrochlorination of allyl chloride
■ alkaline dehydrochlorination of DCH to EPI

An alternative technology developed by Showa-Denko is based on the following steps:
■ Formation of allyl acetate by the catalyzed oxidation of propylene with acetic acid
■ Catalyzed hydrolysis of allyl acetate to allyl alcohol
■ Catalyzed chlorination of allyl alcohol to DCH
■ Alkaline dehydrochlorination of DCH to EPI

Both the above processes are complex, require relatively high investment and elevated consumption of feedstocks, and produce sizable quantities of by products, involving critical effluent problems.

One further process, discovered more than one century ago, is based on the production of DCH by hydrochlorination of GLY in liquid phase with HCl in presence of an homogeneous catalyst.

Since 1870 Reboul discovered that acetic acid catalyzes the reaction of HCl with glycerol.

The reaction proceeds by hydrochlorinating first GLY to monochlorohydrins (MCH), mostly of the α form, which are subsequently converted into DCH, as followingly shown:

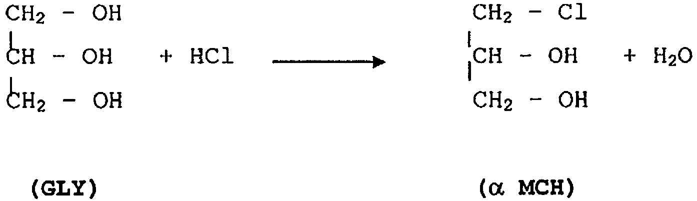

The GLY to MCH reaction goes to completion quite fast.

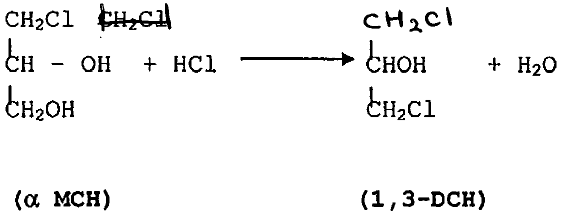

The MCH to DCH reaction is slower, its final equilibrium is affected by the pressure of HCl and by the water present in the reacting mass.

Inferior quantities of 2,3-DCH are formed.

This constitutes an advantage of the process since 1,3-DCH reacts faster than 2,3-DCH in dehydrochlorination, thus forming less by products.

Notwithstanding the simpler chemistry of the process and its potential merit, as the lower investment involved, this technology did not find industrial application due to the high cost of GLY.

It is significant the fact that, for many years, synthetic GLY was produced using EPI as feedstock.

Consequently the GLY to EPI route remained confined in the frame of technical and patent literature.

DE Pat. 197308, 1906 can be considered as representative of the early art.

The above patent describes the reaction between GLY and gaseous hydrogen chloride occurring in liquid phase at about 100°C.

Carboxylic acids are mentioned as adequate catalysts.

Besides acetic acid, other carboxylic acid are mentioned as catalyst, including formic acid, succinic acid, citric acid and propionic acid, the latter being tested and mentioned in one example.

The patent shows that, acting without separation of the formed water, the yield of the reaction in DCH is around 75%.

It does not mention the operating pressure of the reaction, leaving unspecified whether it was atmospheric or higher.

It is common knowledge that operating at higher pressure increases the solubility of HCl in the liquid phase.

The Deutsch-Sprengstoff A.G. DE Pat. 180668 indeed mentions the production of chlorohydrins, particularly MCH, operating the reaction at 120°C under pressure.

Gibson - Chemistry and Industry 1931, pag. 949-954, 970-975 and Conant - Organic Synthesis - Coll. 1941 Vol. 1 pag. 294 reported superior yields of DCH achieved by purging from the reactor a large excess of HCl.

The use of azeotropic agents was suggested.

For instance, U.S. PAT 2,144,612 suggests the addition at an entrainer as di-n-butyl ether, propylene dichloride or chlorobenzene.

The complications of the prior art, such as low yields, high losses of HCl, complex distillations in case of use of entrainers, have contributed to prevent this process from becoming established.

The increasing interest toward the production of biodiesel, which produces GLY as by product, reducing drastically its cost, has stimulated a reconsideration of the GLY to EPI process, in a modern improved version.

WO 2005/021476 suggests to carry the hydrochlorination in from 1 to 5, preferably 3, continuous reaction zones each one associated with continuous removal of the water of reaction by distillation.

The total residence time in the system is from 5 to 40 hr.

All reactors operate at nearly identical pressure.

Tests have been performed at atmospheric pressure, but the patent mentions that pressure may be elevated to improve the solubility of HCl.

The process can use carboxylic acids as catalyst, preferably acetic acid.

French Pat. FR 2862644, Pat. WO 2005/054107 and WO 2006/100312 confirm the removal of water by distillation to improve the yields.

The above patents mentions that the reaction can be carried out under high pressure.

Apart carboxylic acids, substituted benzoic acid derivatives are suggested as catalyst.

Pat. WO 2006/111810 confirms that stripping of water is important to improve the yields.

Pat. WO 2006/020234 describes a process for converting a multihydroxylated aliphatic hydrocarbon (as GLY) to chlorohydrins by contacting GLY with HCl at superatmospheric partial pressure of HCl without substantial removal at water.

Good yields are shown using as catalyst acetic acid, hexanoic acid and certain lactones.

All tests are described in batch operation. The patent mentions the possibility of operating the reaction continuously without giving details of such operation, except for mentioning possible recycle conditions and the need of incorporation in the technology unit operations well known to experienced chemical engineers.

One critical aspect of said process is the fact that the effluent of the reaction will contain, besides the organic matter, water and HCl, the latter in rather large excess being in equilibrium with a vapor phase having a rather high partial pressure of HCl.

This will penalize the economics of the process either in case of direct disposal of water rich in HCl to the dehydrochlorination column, or in case of a installation of complex HCl recovery units.

In order to overcome the above described limit, the present invention provides a process which, besides obtaining high reaction yields and other merits, as better described in the following summary of the invention, permits an optimal use of the hydrogen chloride. Thus, the scope of the invention is defined in the appended claims.

### SUMMARY OF THE INVENTION

This invention relates to a process for the production of DCH, mainly 1,3-DCH, by the catalytic reaction of GLY with HCl.

The process is characterized by using reactors in series, preferably two, operating continuously at different pressures.

The first reactor, low pressure (L.P.) reactor, operating at a pressure ranging from 1 to 4 bar and at a temperature from 90°C to 130°C converts most of GLY to MHC.

The second reactor, medium pressure(M.P.) reactor, operating at a pressure ranging from 5 to 20 bar and at temperature from 90°C to 130°C converts the effluent from the L.P. reactor to DCH with an adequate degree of conversion.

Each reactor is followed by a stripping unit.

The effluent from the L.P. reactor containing, besides water, only minor amounts of HCl and of organics, is stripped at the pressure of the dehydrochlorination column in a L.P. stripping unit in way to make possible the recovery of the heat value of the produced vapour in the dehydrochlorination column itself.

The liquid effluent from the M.P. reactor is stripped at the pressure of the L.P. reactor in a M.P. stripping unit, in way to make possible to recover the HCl contained in the produced vapor, by reacting it with GLY in the L.P. reactor itself.

Most or all the HCl needed in the process is fed to the M.P. reactor, in order to favour the equilibrium to DCH. The excess of HCl, if any, leaving the M.P. reactor in vapor phase is also recycled to the L.P. reactor.

The recycles of vapors from the M.P. reactor to the L.P. reactor constitutes a rather simple and effective method of limiting the losses of HCl in the process.

Furthermore the stripping unit which follows the L.P. reactor, removing a relevant amount of water contained in the effluent, favours the equilibrium of formation of DCH in the following M.P. reactor.

The liquid from the M.P. stripping unit is further stripped in a final stripper operating at the pressure of dehydrochlorination column, in way to make possible an additional recovery of the heat value of the produced vapor, which will contain, besides water, some DCH and only minor amounts of HCl.

The liquid effluent from the final stripper goes to a fractionation column where DCH is recovered as overhead product.

The bottom product, consisting of non converted MCH in combination with various by products, is recycled to the L.P. reactor to improve the yields.

A further merit of this invention is in the possibility of optimizing the process using different conditions in the operation of the L.P. reactor and the M.P. reactor.

In fact, besides the different pressures, it is possible to operate the reactor with different temperatures and even not identical catalysts.

The catalysts employed can be selected among the following:
a) acetic acid and esters of acetic acid with GLY, as mono, di and tri-acetins
b) a carboxylic acid or a compound forming a carboxylic acid at the conditions of the reaction. Such a compound shall have a boiling point above 200°C, a point of decomposition not inferior to 135°C and shall be soluble in GLY and in the organic stream rich in MCH to be recycled to the L.P. reactor.
   Adequate catalyst are, but not limited to, malic acid, succinic acid, glycerol esters of carboxylic acid, heptanoic acid.
c) a combination of the above catalysts where a catalyst of category b) is fed, to the L.P. reactor, while a catalyst of category a) is further added to the M.P. reactor

The following examples are representative of the process, but are not intended to limit the scope of the invention.

The tests have been executed in a 300 ml Hastelloy C reactor provided with a variable speed stirrer, thermocouple for measuring the internal temperature, pressure controller, flow meter on HCl feed line, heating and cooling devices, connection for introducing the feedstocks and purging the system, taking out the product and samples.

The samples were analyzed using gas chromatography analyzer.

### EXAMPLE 1 PREPARATION OF DCH FROM GLY USING ACETIC ACID AS CATALYST

In the reactor have been loaded 150 gr. of GLY containg 6% mol. of acetic acid.

Gaseous HCl was admitted as necessary to maintain a constant pressure of 2 bar at a temperature of 100°C.

After one hour the pressure in the reactor was increased to 9 bar at a temperature of 100°C.

The reaction was let to proceed for additional 3 hr. Samples were taken and analyzed periodically.

The GC analysis, excluding water, catalyst and residual hydrogen chloride, made after each stage of reaction showed the following distribution:

| | AFTER 1 hr | AFTER 4 hr |
|---|---|---|
| GLY | 11. 48 | NEGL. |
| α-MCH | 77.55 | 0.76 |
| β-MHC | 4.94 | 8.08 |
| 1,3-DCH | 6.03 | 89.62 |
| 1,2-DHC | NEGL. | 1.54 |

### EXAMPLE 2 - PREPARATION OF DCH FROM GLY USING MALIC ACID AS CATALYST

The reaction was conducted identical as in example 1, except that 8% mol. of malic acid, referred to the GLY, was used as catalyst.

The GC analysis made after each stage of reaction showed the following distribution:

| | AFTER 1 hr | AFTER 4 hr |
|---|---|---|
| GLY | 7.63 | NEGL. |
| α-MCH | 83.55 | 27.43 |
| β-MHC | 5.65 | 8.20 |
| 1,3-DCH | 3.17 | 63.23 |
| 1,2-DHC | NEGL. | 1.14 |

### EXAMPLE 3 - PREPARATION OF DCH FROM GLY USING MALIC AND ACETIC ACID AS CATALYST

The reaction was conducted similar to example 2, except that an additional 3% mol. of acetic acid, referred to the GLY, was added to the reacting mass after 1 hr time, and after a flash at nearly atmospheric pressure just prior the pressurization.

The reaction was completed in a shorter additional time (2 hr. and 20 minutes instead of the 3 hr. of example 1 and 2).

The GC analysis made after each stage of reaction showed the following distribution:

| | AFTER 1 hr | AFTER 3 hr 20 min. |
|---|---|---|
| GLY | 18.23 | NEGL. |
| α-MCH | 76.40 | NEGL. |
| β-MHC | 5.37 | 4.82 |
| 1,3-DCH | NEGL. | 92.99 |
| 1,2-DHC | NEGL. | 2.19 |

Comparing the results the following points are remarked:
1) Acetic acid is the most active single catalyst allowing high degree of conversion within the specified time.
   However, due to its relatively high volatility, it would be penalized by losses in the vapor streams feeding the dehydrochlorination column.
2) Malic acid is less active catalyst but, as it results from GC analysis, it is very selective.
3) Use of carboxylic acid with high boiling point as catalyst is feasible in case of heavy ends recycle process. In such a process the reaction will produce an amount of unconverted MCH adequate to maintain in solution the catalyst, which, by its recycling to the reaction system, would suffer losses of minor entity.
4) The use of a twin catalyst, as per example 3, is useful to optimize the process being a synthesis of the previous alternatives and allowing to reach in a shorter time higher reaction yields, even superior to the one of pure acetic acid, and lower consumption of catalyst. Surprisingly the use of a twin catalyst, even if malic acid is not the best catalyst in term of reaction rate, allows not only to have the highest activity but also to reach high selectivity, by reducing in particular the production of β-MHC, which is the less reactive intermediate product of reaction.

### BRIEF DESCRIPTION OF THE DRAWING

The distinctive aspect and advantages of process of the present invention will be clearer from the following detailed description, related to the enclosed drawing wherein:

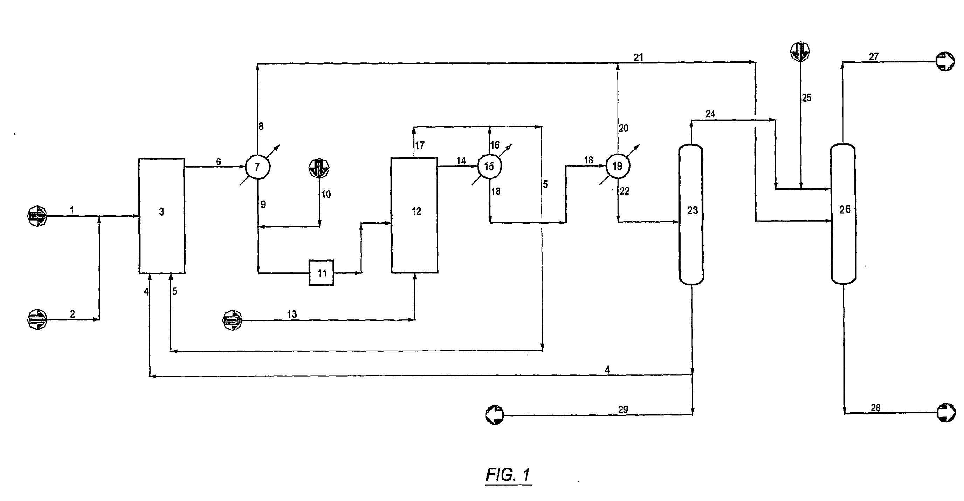

Fig. 1 shows schematically a plant designed according to the process of this invention.

### DETAILED DESCRIPTION OF THE INVENTION

By reference of said Fig.1, the design is based on the use of a combination of carboxylic acid and acetic acid as catalyst.

Feed GLY 1 with a dissolved make-up of carboxylic acid catalyst 2 is fed to a L.P. reactor 3 together with a liquid organic stream 4 formed by non converted MCH, catalyst, chlorohydrin esters and minor by products and with a vapor stream 5 rich in HCl recycled from the M.P. section of the plant.

The effluent 6 from the L.P. reactor is submitted to partial vaporization in a L.P. stripping unit or vaporizer 7.

The vapor stream 8 from the vaporizer 7 is delivered to a dehydrochlorination column 26.

The stripped liquid 9 is mixed with a low boiling catalyst (i.e. acetic acid) 10 and pumped by pump 11 as feedstock to a M.P. reactor 12, where is contacted with a stream of HCl 13 being most or the total amount consumed in the two reactions.

The liquid effluent 14 from the M.P. reactor 12 is submitted to partial vaporization in a vaporizer 15 operating at a pressure slightly higher to that of the L.P. reactor 3.

The vapour 16 leaving the vaporizer joins the vapour 17 leaving the M.P. reactor 12 forming a stream 5 rich in HCl which feeds the L.P. reactor 3.

The liquid 18 leaving the vaporizer is subject to a further vaporization in a final stripper 19.

The vapor 20 leaving the final vaporizer 19 joins the vapor 8 from the L.P. vaporizer 7 and the mixed stream 21 flows to a dehydrochlorination column 26.

The liquid 22 effluent from the final vaporizer 19 flows to a DCH recovery column 23 operating at high vacuum.

The overheads product of this column is the DCH product 24 which, after being mixed with an aqueous slurry or solution of alkali (calcium hydroxide or caustic soda) 25 enters a dehydrochlorination column 26.

The dehydrochlorination column performs in similar way as in the existing conventional technologies, separating overheads a stream of crude epichlorohydrin 27, which is followingly subject to purification to reach high quality standards.

The bottom effluent 28 of the dehydrochlorination column is an aqueous stream containing either calcium chloride or sodium chloride in solution.

The bottom effluent 4 of the DCH recovery column 23 is recycled to the L.P. reactor 3.

A side stream 29 of said effluent 4 is purged away to limit the accumulation in the process of heavy by products.

## Claims

1. A process to produce dichlorohydrin based on the hydrochlorination of glycerine with hydrogen chloride in presence of a catalyst of homogeneous type, with the initial formation of monochlorohydrins, which are subsequently converted into dichlorohydrins, **characterized by** the fact that the reaction is conducted in at least two reactors in series operating continuously at different pressures, wherein:
a) the first reactor is a low pressure (L.P.) reactor which operates at a pressure from 1 to 4 bar and a temperature from 90 to 130°C; while
b) the subsequent reactor is a medium pressure (M.P.) reactor which operates at a pressure from 5 to 20 bar and a temperature from 90 to 130°C.

2. The process of claim 1, **characterized by** the fact that the liquid effluent from the (L.P.) reactor is subjected to partial vaporization and the produced vapor flows directly to a column where, by a process of dehydrochlorination, the produced dichlorohydrins are converted into epichlorohydrin.

3. The process of claims 1 to 2, wherein the liquid effluent from the (M.P.) reactor is subject to two subsequent stages of vaporization; the first stage operating at a pressure close to that of the (L.P.) reactor, produces a stream of vapor containing hydrogen chloride which is recycled to the (L.P.) reactor; the liquid effluent from the first stage is submitted to a second stage of vaporization from where the produced vapor flows directly to the previously defined dehydrochlorination column.

4. The process according to one of claims from 1 to 3, where the liquid effluent from said second stage of vaporization is processed in a fractionating column (DCH Column), which, operating at high vacuum, separates overheads the dichlorohydrin product, while the stream separated at bottom, containing unreacted monochlorohydrins plus other organic by products and catalyst, is recycled to the L.P. reactor.

5. The process according to one claims from 1 to 4, where the catalyst employed in the process is selected from the group consisting of:
a)acetic acid and esters of acetic acid with glycerine, as mono, di and triacetins;
b) a carboxylic acid or a compound forming a carboxylic acid at the conditions of the reaction;
c) a combination of the above catalysts, where a catalyst of category b) is fed to the (L.P.) reactor, while a catalyst of category a) is further added to the (M.P.) reactor.

6. The process according to claim 5 wherein the catalyst of category b) have a boiling point above 200°C, a point of decomposition not inferior to 135°C and is soluble in glycerine and in the organic stream rich in monochlorohydrins to be recycled to the (L.P.) reactor.

7. The process according to one of claims 5 and 6 wherein the catalysts of category b) are selected among malic acid, succinic acid, glycerol esters of carboxylic acids, heptanoic acid.

8. The process according to one of claims from 1 to 5 where the first reactor (L.P. reactor) operates at a pressure from 2 to 3 bar, and at a temperature from 110 to 120°C, being fed with a catalyst of the type described at point b) of claim 5 above, and where the second reactor (M.P. reactor) operates at a pressure from 8 to 15 bar, being fed with a catalyst of the type described at point a) of claim 5 above.

9. The process according to one of claims from 1 to 8, where the produced dichlorohydrins are contacted with an alkaline solution to produce epichlorohydrin in a dehydrochlorination column, such column being fed with vapors produced in the hydrochlorination section of the plant.

10. The process according to one of claims from 1 to 8, where the glycerine feedstock is pure.

11. The process according to one of claims from 1 to 8 where the glycerine feedstock is of the crude type, produced by treatment of natural resources.

## Patentansprüche

1. Verfahren zum Herstellen von Dichlorhydrin auf Basis der Hydrochlorierung von Glycerin mit Chlorwasserstoff in Gegenwart eines Katalysators von homogenem Typ, unter anfänglicher Bildung von Monochlorhydrinen, die nachfolgend zu Dichlorhydrinen umgewandelt werden, **dadurch gekennzeichnet, dass** die Reaktion in wenigstens zwei Reaktoren in Serie durchgeführt wird, die kontinuierlich bei unterschiedlichen Drücken arbeiten, wobei:
a) der erste Reaktor ein Niederdruck- (ND-) Reaktor ist, der bei einem Druck von 1 bis 4 bar und einer Temperatur von 90 bis 130°C arbeitet, während
b) der nachfolgende Reaktor ein Mitteldruck- (MD-) Reaktor ist, der bei einem Druck von 5 bis 20 bar und einer Temperatur von 90 bis 130°C arbeitet.

2. Verfahren gemäß Anspruch 1, **dadurch gekennzeichnet, dass** die flüssige Ableitung aus dem (ND-) Reaktor teilweiser Verdampfung unterzogen wird und der erzeugte Dampf direkt zu einer Kolonne strömt, wo die erzeugten Dichlorhydrine durch ein Dehydrochlorierungsverfahren zu Epichlorhydrinen umgewandelt werden.

3. Verfahren gemäß Anspruch 1 bis 2, wobei die flüssige Ableitung aus dem (MD-) Reaktor zwei nachfolgenden Verdampfungsstufen unterliegt; die erste Stufe, die bei einem Druck in der Nähe desjenigen des (ND-) Reaktors arbeitet, einen Dampfstrom erzeugt, der Chlorwasserstoff enthält, der zu dem (ND-) Reaktor zurückgeführt wird; die flüssige Ableitung aus der ersten Stufe einer zweiten Verdampfungsstufe unterzogen wird, aus welcher der erzeugte Dampf direkt zu der vorangehend bestimmten Dehydrochlorierungskolonne strömt.

4. Verfahren gemäß einem der Ansprüche 1 bis 3, wobei die flüssige Ableitung aus der zweiten Verdampfungsstufe in einer Fraktionierkolonne (DCH-Kolonne) aufbereitet wird, die im Betrieb unter Hochvakuum oben das Dichlorhydrinprodukt abscheidet, während der unten abgeschiedene Strom, der unreagierte Monochlorhydrine plus andere organische Nebenprodukte und Katalysator enthält, zu dem ND-Reaktor zurückgeführt wird.

5. Verfahren gemäß einem der Ansprüche 1 bis 4, wobei der in dem Verfahren eingesetzte Katalysator aus der Gruppe ausgewählt ist, welche besteht aus
a) Essigsäure und Estern der Essigsäure mit Glycerin, als Mono-, Di- und Triacetinen;
b) einer Carbonsäure oder einer Verbindung, die bei den Bedingungen der Reaktion eine Carbonsäure bildet;
c) einer Kombination aus den obigen Katalysatoren, wobei ein Katalysator der Kategorie b) dem (ND-) Reaktor zugeführt wird, während ferner ein Katalysator der Kategorie a) dem (MD-) Reaktor hinzugefügt wird.

6. Verfahren gemäß Anspruch 5, wobei der Katalysator der Kategorie b) einen Siedepunkt von über 200°C, einen Zersetzungspunkt nicht unter 135°C hat und in Glycerin und in dem an Monochlorhydrinen reichen organischen Strom zum Zurückführen zu dem (ND-) Reaktor löslich ist.

7. Verfahren gemäß einem der Ansprüche 5 und 6, wobei die Katalysatoren der Kategorie b) ausgewählt sind aus Äpfelsäure, Bernsteinsäure, Glycerolestern von Carbonsäuren, Heptansäure.

8. Verfahren gemäß einem der Ansprüche 1 bis 5, wobei der erste Reaktor (ND-Reaktor) bei einem Druck von 2 bis 3 bar und bei einer Temperatur von 110 bis 120°C arbeitet, wobei er mit einem Katalysator des oben bei Punkt b) aus Anspruch 5 beschriebenen Typs gespeist wird, und wobei der zweite Reaktor (MD-Reaktor) bei einem Druck von 8 bis 15 bar arbeitet, wobei er mit einem Katalysator des oben bei Punkt a) aus Anspruch 5 beschriebenen Typs gespeist wird.

9. Verfahren gemäß einem der Ansprüche 1 bis 8, wobei die erzeugten Dichlorhydrine mit einer alkalischen Lösung in Berührung gebracht werden, um in einer Dehydrochlorierungskolonne Epichlorhydrin zu erzeugen, wobei diese Kolonne mit Dämpfen gespeist wird, die in dem Hydrochlorierungsabschnitt der Anlage erzeugt werden.

10. Verfahren gemäß einem der Ansprüche 1 bis 8, wobei das Glycerin-Ausgangsmaterial rein ist.

11. Verfahren gemäß einem der Ansprüche 1 bis 8, wobei das Glycerin-Ausgangsmaterial vom rohen Typ ist, der durch Behandlung natürlicher Rohstoffe erzeugt ist.

## Revendications

1. Procédé de production de dichlorhydrine basé sur la chlorohydration de glycérine avec du chlorure d'hydrogène en présence d'un catalyseur de type homogène, avec la formation initiale de monochlorhydrines, qui sont ensuite converties en dichlorhydrines, **caractérisé par le fait que** la réaction est opérée dans au moins deux réacteurs en série fonctionnant en continu à des pressions différentes, dans lequel :
a) le premier réacteur est un réacteur basse pression (B.P.) qui fonctionne à une pression de 1 à 4 bars et à une température de 90 à 130°C ; alors que
b) le réacteur suivant est un réacteur à pression moyenne (P.M.) qui fonctionne à une pression de 5 à 20 bars et à une température de 90 à 130°C.

2. Procédé selon la revendication 1, **caractérisé par le fait que** l'effluent liquide provenant du réacteur (B.P.) est soumis à une vaporisation partielle et la vapeur produite s'écoule directement vers une colonne où, par le biais d'un procédé de déchlorhydratation, les dichlorhydrines produites sont converties en épichlorhydrine.

3. Procédé selon les revendications 1 à 2, dans lequel l'effluent liquide provenant du réacteur (P.M.) est soumis à deux étages consécutifs de vaporisation ; le premier étage, fonctionnant à une pression proche de celle du réacteur (B.P.), produit un flux de vapeur contenant du chlorure d'hydrogène qui est recyclé vers le réacteur (B.P.) ; l'effluent liquide provenant du premier étage est soumis à un second étage de vaporisation à partir duquel la vapeur produite s'écoule directement vers la colonne de déchlorhydratation préalablement définie.

4. Procédé selon l'une des revendications 1 à 3, dans lequel l'effluent liquide provenant dudit second étage de vaporisation est traité dans une colonne de fractionnement (colonne DCH), qui, fonctionnant à un vide élevé, sépare dans la partie supérieure le produit de dichlorhydrine, alors que le flux séparé dans la partie inférieure, contenant des monochlorhydrines n'ayant pas réagi en plus d'autres sous-produits organiques et d'un catalyseur, est recyclé vers le réacteur B.P.

5. Procédé selon l'une des revendications 1 à 4, dans lequel le catalyseur employé dans le procédé est choisi dans le groupe constitué par :
a) l'acide acétique et les esters d'acide acétique avec glycérine, sous la forme de mono, di et triacétines ;
b) un acide carboxylique ou un composé formant un acide carboxylique aux conditions de la réaction ;
c) une combinaison des catalyseurs ci-dessus, où un catalyseur de catégorie b) est fourni au réacteur (B.P.), alors qu'un catalyseur de catégorie a) est en outre ajouté au réacteur (P.M.).

6. Procédé selon la revendication 5, dans lequel le catalyseur de catégorie b) a un point d'ébullition au-dessus de 200°C, un point de décomposition pas inférieur à 135°C et est soluble dans de la glycérine et dans le flux organique riche en monochlorhydrines devant être recyclé vers le réacteur (B.P.).

7. Procédé selon l'une des revendications 5 et 6, dans lequel les catalyseurs de catégorie b) sont choisis parmi l'acide malique, l'acide succinique, les esters de glycérol d'acides carboxyliques, l'acide heptanoïque.

8. Procédé selon l'une des revendications 1 à 5, dans lequel le premier réacteur (réacteur B.P.) fonctionne à une pression de 2 à 3 bars, et à une température de 110 à 120°C, étant alimenté avec un catalyseur du type décrit au point b) de la revendication 5 ci-dessus, et dans lequel le second réacteur (réacteur P.M.) fonctionne à une pression de 8 à 15 bars, étant alimenté avec un catalyseur du type décrit au point a) de la revendication 5 ci-dessus.

9. Procédé selon l'une des revendications 1 à 8, dans lequel les dichlorhydrines produites sont mises en contact avec une solution alcaline pour produire une épichlorhydrine dans une colonne de déchlorhydratation, une telle colonne étant alimentée avec les vapeurs produites dans la section de chlorhydration de l'installation.

10. Procédé selon l'une des revendications 1 à 8, dans lequel la charge de glycérine est pure.

11. Procédé selon l'une des revendications 1 à 8, dans lequel la charge de glycérine est du type brut, produite par traitement de ressources naturelles.
